# EUROPÄISCHE PATENTANMELDUNG

(11) **EP 0 531 955 A2**
(43) Veröffentlichungstag der Anmeldung: **17.03.1993**
(21) Anmeldenummer: 92115377.1
(22) Anmeldetag: 09.09.1992
(51) Int. Cl.: G01N 33/18, C02F 3/00

(54) **Verfahren und Vorrichtung zur Bestimmung der Nitrifikationsaktivität in einem Abwasserstrom oder -becken und deren Anwendung**

(30) Priorität: 13.09.1991 DE 4130465
(71) Anmelder: FORSCHUNGSZENTRUM JÜLICH GMBH, D-52425 Jülich (DE)
(72) Erfinder: Aivasidis, Alexander, Dr., W-5170 Jülich (DE); Wandrey, Christian, Prof., W-5170 Jülich (DE)

(57) **Zusammenfassung**

Die Nitrifikationsaktivität in einem Abwasserstrom oder -becken wird zweckmäßigerweise mittels eines Abwasserteilstroms überwacht, der mit definiertem pH-Wert und bestimmter Verweilzeit durch eine als Bioreaktor ausgebildete DurchfIußzelle geschickt wird, die eine Nitrifikantenpopulation aufweist oder zusammen mit dem Abwasser erhält. Gemessen wird die pH-Korrekturmittelmenge, die notwendig ist um die mit der Nitrifikation verbundene pH-Änderung zu kompensieren. Zusätzlich kann die Trübung in der DurchfIußzelle ermittelt werden. Je nach Aufgabenstellung kann mit NH₄⁺-Standardlösung die zu ermittelnde Nitrifikationsaktivität einer zu bewertenden Bakterienpopulation oder die Ammoniumbelastung mittels einer Population bekannter Aktivität ermittelt werden oder aber Adaptionsgrößen bestimmt werden, die für vorhandene Abwasserlast und Klärschlammaktivitäten erforderlich sind. Das Verfahren kann insbesondere für die Zulaufregulierung zu einem Nitrifikationsreaktor benutzt werden. Als miniaturisierte Bioreaktoren ausgebildete Durchflußzellen mit geträgerter Biomasse, Meßsonden und Dosierzulauf für Prüflösung und pH-gesteuertem pH-Korrekturmittelzulauf mit elektronischer Auswert- und Steuereinheit können standardisiert angeboten werden.

## Beschreibung

Die Erfindung bezieht sich auf ein Verfahren zur kontinuierlichen Überwachung der volumenbezogenen Nitrifikationsaktivität in einem Abwasserstrom oder -becken sowie auf eine dafür geeignete Vorrichtung und deren Anwendung in (Ab)Wasserreinigungsanlagen.

Neben der Elimination von Kohlenstoffverbindungen aus Abwässern gewinnt heutzutage die Entfernung von Stickstoff immer mehr an Bedeutung. Von den diversen avisierten Verfahren zur Entfernung von Ammonium-Stickstoff aus industriellen sowie landwirtschaftlichen und kommunalen Abläufen ist die biologische Variante besonders interssant, weil nur dadurch eine echte Entsorgung stattfindet, während bei den physikalischen bzw. physikalisch-chemischen Methoden (Strippen, Adsorption/Ionenaustausch, Oxidation mit Hypochlorid) lediglich eine Verlagerung der Abwasserproblematik bzw. sogar eine Verschärfung der Probleme eintritt.

Die biologische Ammoniumelimination besteht aus zwei Schritten, einem unter oxischen Bedingungen ablaufenden (aeroben) Nitrifikationsschritt, bei dem Ammonium unter Beteiligung von Mikroorganismen der Gattung Nitrosomonas und Nitrobacter zu Nitrit und Nitrat oxidiert wird, und einem anoxische Bedingungen erfordernden (anaeroben) Denitrifikationsschritt, bei dem die gebildeten Oxidationsprodukte durch heterotrophe Nitratatmer bei gleichzeitiger Verwertung einer Kohlenstoffquelle (Elektronendonator) zu molekularem Stickstoff (N₂) reduziert werden.

Der erste Teilschritt der Nitrifikation stellt dabei mit mindestens zwei Mikroorganismenarten, welche durch lange Generationszeiten und eine ausgeprägte Energielimitierung des Wachstums aufgrund des besonders energieintensiven CO₂-Fixierungsprozesses gekennzeichnet sind, den geschwindigkeitsbestimmenden Schritt im Zuge der Ammoniumelimination dar. Unter diesem Aspekt wird die Notwendigkeit umso deutlicher, eine kontinuierliche Diagnose der biologischen Aktivität vorzunehmen und eine Hemmung der Nitrifikation möglichst verzögerungsfrei zu identifizieren.

Mikroorganismen reagieren in bezug auf ihre Abbauleistung im allgemeinen sehr empfindlich auf Schwankungen der Raumbelastung der Abwasserinhaltstoffe, der Versorgung mit Nährstoffen und Spurenelementen (wobei insbesondere bei aeroben Prozessen die Sauerstoffversorgung hervorzuheben ist) sowie das Vorkommen toxischer Komponenten, so daß für solche Prozesse eine zuverlässige Regelung besonders wichtig ist. Diese impliziert wiederum im Om-line-Modus gewonnene Prozeßinformationen.

Die Steuerung biotechnologischer Prozesse hat im den letzten 15 Jahren signifikant an Bedeutung gewonnen. Nicht zuletzt hat die Entwicklung moderner digitaler Prozeßrechner für einen erheblichen Fortschritt auf diesem Gebiet gesorgt. Die Nutzung moderner digitaler Prozeßtechnik konnte allerdings in noch stärkerem Maße als bislang in den biologischen Prozeß integriert werden, wenn mehr als bisher prozeßrelevante Meßgrößen on-line erfaßt werden könnten. D.h., zur Verbesserung der Verfahrenstechnik bei der biologischen Abwasserreinigung ist zunächst eine Verbesserung der Analytik, insbesondere die Entwicklung von kontinuierlichen Meßtechniken, wichtig.

Da die Leistungsfähigkeit und Reinigungseffizienz einer biologischen Kläranlage in direkter Beziehung zur Stoffwechselaktivität der in Frage kommenden Mikroorganismen steht, spielt deren kontinuierliche Erfassung eine ganz erhebliche Rolle.

Die On-line-Messung biologischer Parameter für die Stoffwechselaktivität ist in der Praxis sehr problematisch. Bislang beschränkt sich die Automatisierung von Kläranlagen daher im allg. auf die Einhaltung einer vorgegebenen Konzentration des Gelöst-Sauerstoffs und eines bestimmtem pH-Wertes sowie die Einstellung eines "Schlammalters" durch partielle Überschußschlamm-Rückführung im das Belebungsbeckem.

Zwar gibt es bereits Geräte (z.B. Rodtox® und Stiptox®) zur quasi-kontinuierlichen Kurzzeit-BSB-Messung (Bestimmung des biochemischen Sauerstoffbedarfs), die nach entsprechender Modifizierung auch als Bakterientoximeter geeignet sind. In beiden Fällen wird der Sauerstoffverbrauch durch die mikrobielle Umsetzung organischer Bestandteile und seine Konzentrationsänderung mittels einer polarographischen Elektrode gemessen und durch Prozeßrechner-Auswertung zum Gelöst-BSB bzw. zur auftretenden Toxität in Relation gesetzt. Eine kontinuierliche Aufzeichnung der Mikroorganismenaktivität ist in beiden Fällen weder für die Kohlenstoff- noch die Stickstoffelimination möglich. Aber auch die Indikation einer Nitrifikationshemmung kann auf diese Weise nicht erzielt werden, weil
- nitrifizierende Mikroorganismen nicht durch die gleichen Reagenzien wie die Heterotrophen gehemmt werden und vice versa und
- eine Differenzierungsmöglichkeit allein über die Messung des Summenparameters Sauerstoff nicht möglich ist.

Zwar wird der BSB bei gleichzeitigem Vorliegen von Ammonium durch gezielte Hemmung der Nitrifikation mittels Allylthioharnstoff im diskontinuierlichen Meßverfahren gesondert ermittelt. Eine daraus ableitbare gesonderte Bestimmung der Nitrifikationsaktivität ist jedoch nicht bekannt.

Der Erfindung liegt daher im wesentlichen die Aufgabe zugrunde, ein geeignetes Meßverfahren für die kontinuierliche Bestimmung der Aktivität nitrifizierender Mikroorganismen vorzusehen, mit dem deren Stoffwechselaktivität in einem Abwasserreinigungssystem und/oder die Belastung des Zulaufs ermittelt werden kann und ggfs. toxische Komponenten auf der Basis einer on-line ermittelten Substratverbrauchsgeschwindigkeit erkannt werden können.

Das zur Lösung dieser Aufgabe entwickelte erfindungsgemäße Verfahren ist im wesentlichen dadurch gekennzeichnet, daß man kontinuierlich einen Abwasserteilstrom von definiertem pH-Wert mit bestimmter Verweilzeit durch eine als Bioreaktor ausgebildete, durch Zufuhr von pH-Korrekturmittellösung pH-kontrollierte Durchflußzelle schickt, die ggfs. mit angepaßter NH₄⁺-Standardlösung simultan oder alternativ gespeist wird und eine Nitrifikantenpopulation aufweist oder zusammen mit dem Abwasser erhält und daß man die zugesetzten pH-Korrekturmittelmengen fortlaufend mißt und in Signale der Nitrifikationsleistung umsetzt.

Wesentliches Element dieses Verfahrens ist die Verwendung einer als Bioreaktor ausgebildeten Durchflußzelle, durch die ein repräsentativer Probenstrom geleitet wird, der (a) bei Herkunft aus einer biologischen Klärstufe Nitrifikanten enthalt, deren Aktivität im orginär vorliegenden System ermittelt werden soll. Alternativ stammt der Probenstrom (b) vom Abwasserzulauf zur Nitrifikationsstufe und soll auf seine Belastung und ggfs. das Vorliegen von Hemmstoffen geprüft werden, weshalb entweder biomassehaltiger Ablauf der Klärstufe zugemischt oder (ggfs. adaptierte) insb. geträgte Nitrifikanten in der Zelle vorgesehen werden.

Im Falle (a) wird (insb. im Falle praktisch vollständiger Nitrifikation der Stickstoffverbindungen in der Klärstufe) ständig oder wiederkehrend NH₄⁺-haltige Standardlösung vor oder in der Zelle zugemischt, so daß über die Umsetzung der bekannten Lösung eine Bewertung der Nitrifikationsleistung der Reinigungsstufe möglich ist. Im Falle (b) liegt im Wasser im allg. genügend Ammoniumstickstoff vor, so daß bei bekannter Biomasseaktivität (insb. der Organismen der Nitrifikationsstufe oder mittels gesondert angezüchteter und ggfs. adaptierter Mikroorganismen) die erforderliche Nitrifikationsaktivität zur ausreichenden Nitrifikation im Wasser ermittelt werden kann. Zusätzlich kann auch im Falle (b) die (alternierende) Zumischung einer NH₄⁺-Standardlösung zweckmäßig sein, um die Anwesenheit von Nitrifikations-Hemmwirkungen zu erkennen.

Werden im Zulauf Nitrifikations-Hemmwirkungen erkannt, so ist es zweckmäßig, den Abwasserstrom einem (üblicherweise nicht gefüllten) Speicherbecken zuzuleiten, dessen Kapazität ausreicht, kurzzeitige Störwirkungen durch ausreichende Verdünnung zu "entschärfen", bevor ein Kontakt des Wassers mit den Nitrifikanten der Klärstufe erfolgt und/oder ggfs. eine Analyse und gezielte Beseitigung des Störfaktors vorzunehmen.

In der Durchflußzelle selbst werden die der Biomasse angemessenen physiologischen Größen wie Sauerstoffkonzentration, Temperatur, pH-Wert, Nährstoffversorgung etc. aufrechterhalten.

Als Meßgröße für die volumenbezogene mikrobielle Aktivität wird die zur Aufrechterhaltung eines vorgegebenen pH-Werts (im allg. 7 oder 7,5) zugesetzte pH-Korrekturmittelmenge fortlaufend registriert bzw. bestimmt.

Diesem Verfahren liegt die Erkenntnis zugrunde, daß bei der biologischen Ammoniumoxidation pro mol umgesetzten Stickstoffs 2 mol Protonen gebildet werden. Hieraus resultiert ein signifikanter pH-Abfall, welcher zur Gewährleistung einer optimalen Nitrifikationsaktivität mit Lauge kompensiert werden muß. Pro mg umgesetzten Ammomiumstickstoffs entstehen somit 0,143 mg Protonen, die es abzufangen gilt. Auf 1 l wässriger Lösung bezogen entspricht diese Menge wiederum einer Konzentration von 1,43 x 10⁻⁴ mol/ℓH⁺. Würde also in einem ungepufferten System, ausgehend von einem neutralen pH-Wert, 1 mg NH₄⁺-N nitrifiziert, so ergäbe sich ein pH-Wert von ca. 3,8. Werden nun die aus der NH₄⁺-Oxidation resultierenden Protonen mit einem pH-Korrekturmittel definierter Konzentration titriert, dessen Verbrauch zeitlich erfaßt wird, läßt sich direkt auf die im vorliegenden Volumen pro Zeiteinheit umgesetzte Ammoniummenge und somit auf die volumenbezogene Aktivität der Nitrifikanten schließen.

Zweckmäßigerweise verwendet man zur pH-Einstellung statt üblicher Basen wie z.B. Natronlauge Natriumcarbonat, um von vornherein eine mögliche CO₂-Wachstumslimitierung der autotrophen Nitrifikanten zu vermeiden.

Zusätzlich kann die Biomassekonzentration mittels einer Trübungsmeßsonde ermittelt und so die spezifische Aktivität der Nitrifikanten im Abwasser online berechnet werden, was allerdings voraussetzt, daß keine weiteren Feststoffe neben der Biomasse in der Flüssigkeit vorliegen.

Die Auswertung der ermittelten Meßgrößen erfolgt vorzugsweise mit Hilfe eines Prozeßrechners, der die Meßdaten aus pH-Regler, "Korrekturmittelzähler" (digitaler Chronograph mit Additiomszählwerk für die Erfassung der Impulslängenzeiten der Korrekturmittelzugabe) und ggfs. der Trübungsmeßsonde komtinuierlich erfaßt und auswertet. Aus der mittels des Zeitzählers über eine optimierte Zeitspanne von z.B. 30 min ermittelten Impulslängen-Gesamtdauer der Korrekturmittelzufuhr und einer entsprechenden Pumpeneichung kann vom Prozeßrechner die pro Zeiteinheit eingetragene Korrekturmittelmenge und daraus die volumenbezogene Aktivität berechnet werden.

Die Biomassekonzentration kann z.B. gleichzeitig als Mittelwert über 15 Meßwerte der Trübung ermittelt werden und zur Umrechnung in spezifischen Biomasseaktivität dienen, die allerdings lediglich als eine komplementäre Größe betrachtet werden kann, da im allgemeinen die kontinuierliche Erfassung der volumembezogenen mikrobiellen Aktivität für die Steuerung der Nitrifikation vollkommen genügt.

Die Verweilzeiten in der Zelle liegen zweckmäßigerweise bei 0,5 bis 2 Stunden.

Die NH₄⁺-Standardlösung kann jede den Verhältnissen angemessene Konzentration von z.B. zwischen 20 und 200 mmol NH₄⁺/l und darüber aufweisen. Üblicherweise ist eine NH₄⁺-Standardlösung mit 80 mmol NH₄⁺ pro Liter geignet, deren pH-Wert dem pH-Wert des Probenstroms entsprechen soll.

Weitere Besonderheiten der Erfindung gehen aus den Patentansprüchen und aus der nachfolgenden Beschreibung von Beispielen anhand der beigefügten Zeichnungen hervor. Es zeigen:
- Figur 1: Eine Durchflußzelle zur Realisierung des erfindungsgemäßen Meßprinzips im Schema;
- Figur 2: die Anordnung einer solchen Durchflußzelle bei einer Abwasserreinigungsamlage mit vorgeschaltetem Speicherbecken;
- Figur 3: eine miniaturisierte Meßzelle mit geträgerter Biomasse bekannten Aktivität als Biosensor für den Ammonium- und Hemmstoffgehalt im Abwasserzulauf zur Reinigungsanlage;
- Figur 4 bis 6: Kurven für die Nitrifikationsaktivität bei Zugabe unterschiedlicher Hemmstoffe und
- Figur 7: das Ergebnis eines Vergleichsversuchs mit und ohne Aufstau des zu reinigenden Wassers bei vorübergehenden Phenoleinbruch.

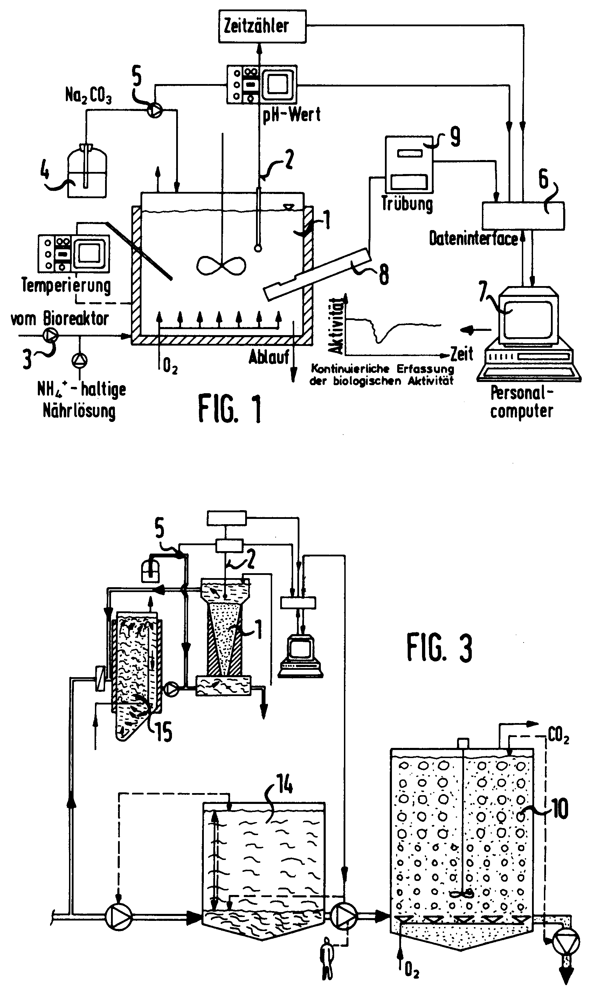

Das in Fig. 1 gezeigte Fließschema für ein Meßverfahren mit prozeßrechnergestützter On-line-Meßtechnik und -Auswertung umfaßt eine Durchflußzelle 1 mit einer pH-Meßsonde 2, in die gemäß vorliegenden Beispiel ein vom Bioreaktor herkommender Probenstrom über die Pumpe 3 eingeleitet wird. Mittels der pH-Meßsonde 2 wird die Zugabe von pH-Korrekturmittel 4 über die Pumpe 5 gesteuert, von der aus eine Zeitmengenangabe zum Interface 6 gelangt. Über den Personal Computer 7 erfolgt die Umrechnung der erhaltenen Meßwerte in Werte für die Nitrifikationsaktivität. Zusätzlich können über eine Sonde 8 und Meßgerät 9 Daten für die Biomassekonzentration über die Trübungsmessung ermittelt und an das Interface weitergegeben werden.

Dem vom Bioreaktor über die Pumpe 3 herkommenden biomassehaltigen Abwasserstrom wird NH₄⁺-haltige Standardlösung zudosiert (Nährlösung), deren bekannter NH₄⁺-Gehalt eine Bewertung der Biomasse des über 3 zugeführtem Stroms ermöglicht.

Dabei kann die Standardlösung entweder ständig zugemischt werden (insb. wenn in der vom Bioreaktor herkommenden Lösung aufgrund der Aktivität der Biomasse bereits vollständig nitrifiziertes Material zugeliefert wird) oder aber die Zufuhr der Standardlösung erfolgt schubweise, so daß im Abwasser enthaltene Ammoniumreste und/oder vorhandene Hemmstoffe erkannt werden können.

Die Ermittlung von Nitrifikanten-Hemmstoffen ist insb. im Zulauf zu einem Reinigungsreaktor wichtig, weshalb vorzugsweise abwechselnd ein Probenstrom des Zulaufs sowie ein Probenstrom des Ablaufs in der genannten Weise durch die Durchflußzelle geschickt wird.

Bei der praktischen Anwendung dieser Meßanordnung wurde der kontinuierlich aus dem Bioreaktor entnommene Teilstrom (ca. 250 ml/h) mit dem gleichen Volumen einer NH₄⁺ -haltigen Nährlösung identischen pH-Wertes (z.B. 7,6) vermischt und der Durchflußzelle zugeführt. Bei einem Gesamtflüssigvolumen von 0,5 l betrug die Verweilzeit 1 Stunde, so daß gegenüber der Verweilzeit im Abwasserreaktor von der Größenordnung 12-24 Stunden ein Rückgang der Nitrifikationsaktivität mit geringer Verzögerung detektiert werden kann. Die optimale Ammoniumkonzentration der zugeführten Lösung wurde aus Vorversuchen ermittelt und betrug 80 mmol NH₄⁺/l. Darüber hinausgehende Konzentrationen führten zu keiner signifikanten Erhöhung der Nitrifikationsaktivität. Deutlich geringere Konzentrationen sind ebenfalls nicht vorteilhaft, weil dann nur eine verhältnismäßig schwache Resonanz des biologischen Systems resultiert.

Die kontinuierliche Zuleitung einer angemessenen Ammoniumkonzentration war im vorliegendne Fall erforderlich, da in der Belebungsanlage das Ammonium zielgemäß bis auf wenige mg/l umgesetzt wurde und somit seine Ablaufkonzentration für die Gewinnung signifikanter Daten in der geschilderten Meßanordnung nicht ausreichten. Um darüber hinaus eine mögliche Limitierung durch weitere Nährstoffe sowie Spurenelemente zu vermeiden, wurde dem gleichen Flüssigkeitsstrom eine entsprechend angesetzte optimierte mineralische Nährlösung supplementiert.

Die erfindungsgemäße Ermittlung der Nitrifikationsaktivität wurde unter ausgewählten Prozeßbedingungen mit simulierter Störung angewandt. Solche Störungen können auf Schwermetallionen komplexierende Agentien, wie z.B. Allyltioharnstoff oder auf organische Verbindungen wie z.B. Phenol oder Essigsäure zurückgehen, die durch Erhöhung des Gelöst-CSB zu einem Selektionsvorteil der mit den Nitrifikanten um den Sauerstoff konkunierenden Heterotrophen führen.

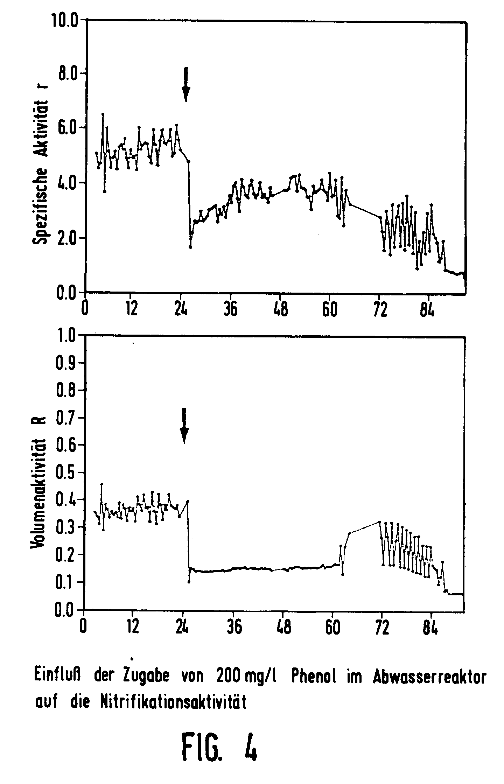

Die beigefügten Figuren 4 bis 6 zeigen den jeweiligen Einfluß der Hemmstoffzugabe (markiert durch den dicken Pfeil) auf den Kurvenverlauf.

Wie diese Figuren zeigen, kann durch gleichzeitige Bestimmung der Biotrockenmassekonzentration die Art des zugesetzten Hemmstoffs erkannt werden: Während die Aktivität der Nitrifikanten durch Allyltioharnstoff praktisch zum erliegen kommt, ohne daß sich die BiomasseKonzentration wesentlich ändert, wird durch Zugabe von Essigsäure die Nitrifikationsleistung auf Null herabgesetzt, während gleichzeitig ein steiler Anstieg der Biomassekonzentration zu verzeichnen ist. Durch Phenol wird ein Abfall der Nitrifikationsleistung verursacht, die sich auf ein mittleres Niveau einpendelt.

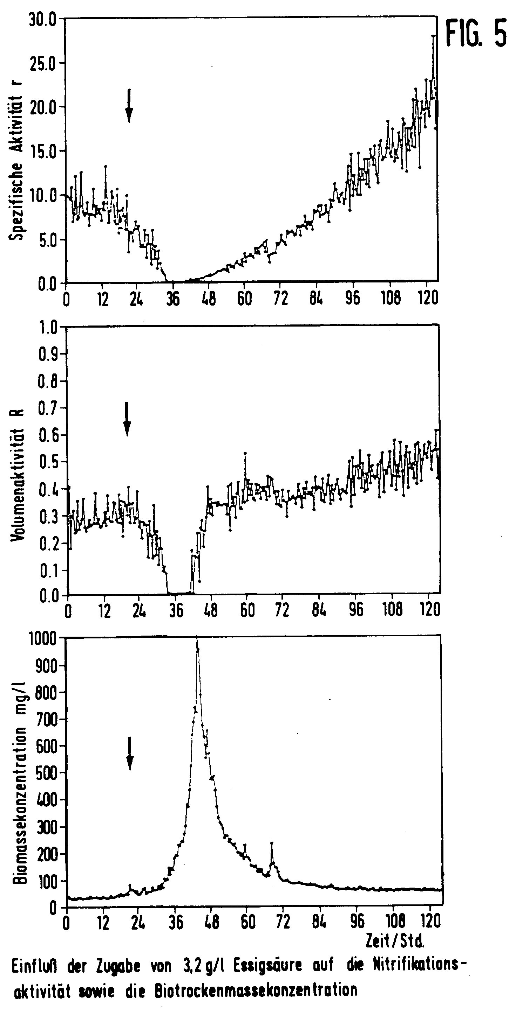

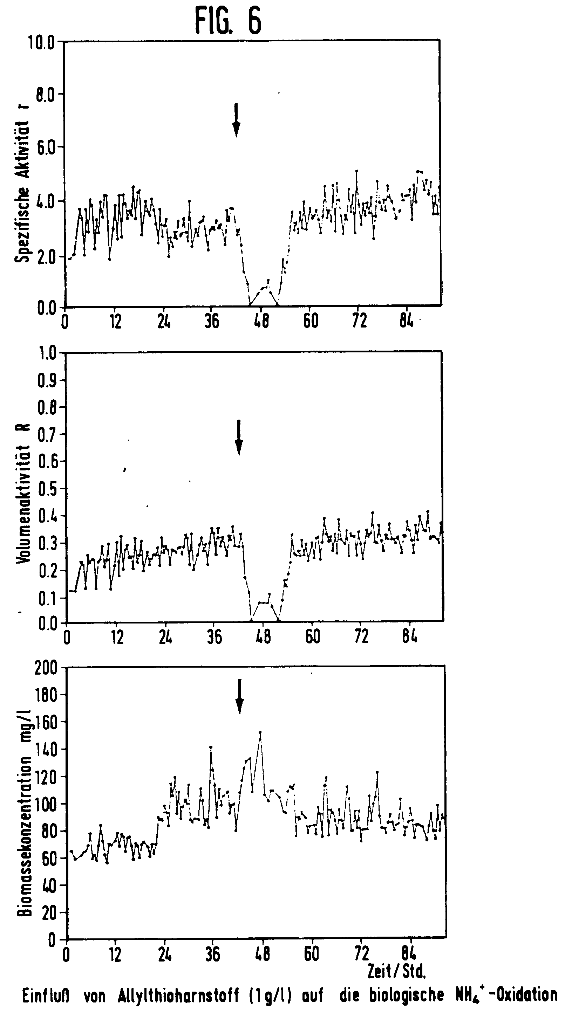

Bei diesen Versuchen wurde die Nitrifikation in einem 10 l Reaktor durchgeführt, dem zu den jeweils angegebenen Zeitpunkten 200 mg/l Phenol (Figur 4), 3,2 g/l Essigsäure (Figur 5) bzw. 1 g/l Allyltioharnstoff (Figur 6) zugesetzt wurden.

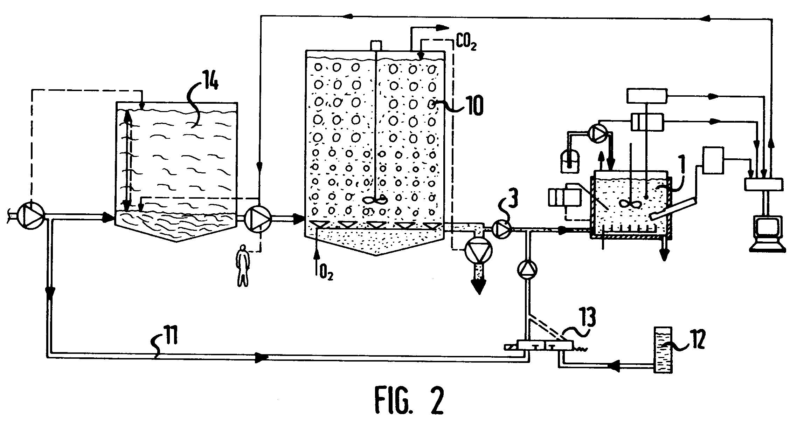

Figur 2 zeigt nun eine Anordnung, bei der die Zelle 1 (mit Zubehör 2 bis 9 wie in Figur 1) mit Ablauf eines Nitrifikationsreaktors 10 über die Pumpe 3 gespeist wird oder alternativ bzw. zugemischt Abwasserzulauf über 11 bzw. NH₄⁺-Standardlösung 12 erhält. Das über eine Zeitschaltuhr angesteuerte Magnetventil 13 sorgt für die alternative Zumischung von NH₄⁺-haltiger Nährlösung oder Rohabwasserzulauf zur Durchflußzelle 1. Durch eine solche kombinierte Fahrweise kann eine Änderung der Belastung bzw. toxische Störung bereits zu einem sehr frühen Zeitpunkt - lange bevor die Population im Reaktor signifikant durch toxische Komponenten beeinflußt wird - erfaßt und die weitere Abwasserzufuhr in den Belebtschlammreaktor aus dem bis dahin mit geringer Flüssigkeitshöhe betriebenen Eingangsbehälter 14 gestoppt werden. In der darauffolgenden Zeit und bis zur Beseitung der Störung wird das ankommende Abwasser im nun als Speicher dienenden Eingangsbehälter 14 aufgestaut und erst nach hinreichend hoher Verdünnung der Schadstoffe erneut durch die Anlage geleitet.

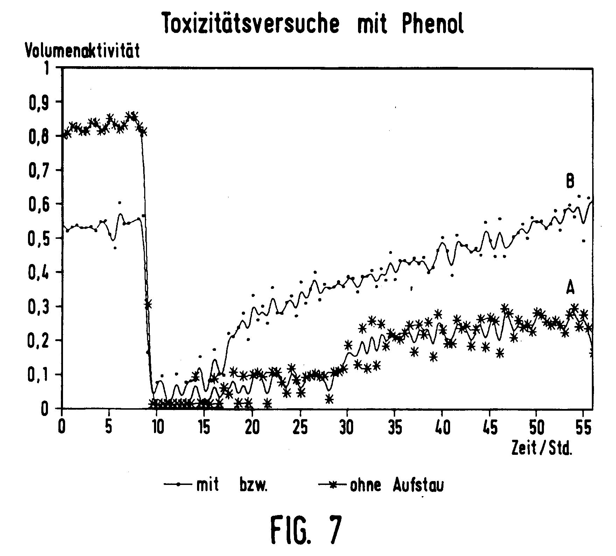

Figur 7 zeigt das Ergebnis eines Vergleichsversuchs: Gemessen wurde die Nitrifikationsaktivität einer Mischung von Zu- und Ablauf eines 45ℓ-Nitrifikationsreaktors mit bei A und B etwas unterschiedlicher Biomassedichte, wobei im den Abwasserstrom zum 20ℓ-Eingangsbehälter (mit 5 - 10 % Füllung) nach 9 Std. jeweils 3 Std. lang 0,5 g/l Phenol zugesetzt wurden.

Dies führte im Falle A (ohne Aufstau im Speicher) zu einer starken Verminderung der Nitrifikationsaktivität im Reaktor, die auch nach längerer Zeit nicht wieder auf den Ursprungswert ansteigt.

Im Falle B wurde von einer vorgeschalteten Durchfluß-Meßzelle das Auftreten von Hemmstoff im Zufluß erkannt und die Verbindung zwischen Nitrifikationsreaktor 1 und Speicherbehälter 14 für 6 Std. unterbrochen. Danach wurde 18 Std. lang Abwasser mit halbem Durchsatz an den Nitrifikationsreaktor weitergegeben, der anschließend 24 Std. lang zur "Entleerung" des Speicherbehälters mit erhöhtem Durchsatz beaufschlagt wurde.

Wie man sieht, erreicht die Aktivität im Nitrifikationsreaktor beim Versuch B danach praktisch wieder ihren ursprünglichen Wert.

Eine speziell auf die Möglichkeit der "Vorprüfung" abgestellte Anordnung ist in Figur 3 wiedergegeben: Hier ist die Durchflußzelle als miniaturisierter Festbett-oder Wirbelschichtreaktor mit hochverdichteter Biomasse ausgebildet, mit dem die biologische Abbaubarkeit der im zufließenden Abwasserstrom vorliegenden Bestandteile in Vorfeld getestet und toxische Stöße frühzeitig erkannt werden können.

In dieser Ausführung ist der Biosensor nicht nur für den Bereich der ( aeroben ) Nitrifikation geeignet, sondern auch darüber hinaus für einem Einsatz unter (anaeroben) Bedingungen, welche bei der Methanisierung oder Denitrifikation vorliegen. Da die Bildung von Methan durch mikrobielle Zersetzung organischer Bestanteile zum überwiegenden Teil auf den Abbau von Essigsäure zurückzuführen ist und dadurch eine Umsatz-abhängiger pH-Anstieg resultiert, kann ein derartiger Biosensor auch zur Vorprüfung in einer Biogasanlage eingesetzt werden. Im Falle der Denitrifikation werden pro mol umgesetzten NOₓ⁻ ca. 0,25 mol Hydroxylionen gebildet.

Eine hohe Biomassedichte bei gleichzeitiger Minimierung der Auswaschrate wird dadurch erreicht, daß die Mikroorganismen an makroporösen dreidimensional kolonisierbaren Trägermaterialien immobilisiert sind. Hierbei kann es sich sowohl um dieselbe Mikroorgansmenpopulation handeln, welche auch im eigentlichen Abwasserreinigungsreaktor vorkommt, oder auch um eine definierte, insbesondere auf den Abbau einzelner Abwasserbestandteile separat adaptierte Biologie. Die erste Möglichkeit hat den Vorteil, daß die Verhältnisse im Vorprüfer und im "Großreaktor" nahezu identisch sind und die Mikroorganismen bei beiden Systemen in gleicher Weise gegenüber einer Intoxifikation reagieren, jedoch muß das für den Vorprüfer erforderliche Immobilisat "on-line" gewonnen werden.

Dem gegenüber kann bei der zweiten Variante die Immobilisierungsmatrix (einschließlich Biologie) in gefriergetrockneter oder tiefgefrorener, wiederbelebbarer Form beliebig verfügbar sein. Hierbei besteht allerdings das potentielle Risiko, daß aufgrund der ungügenden Adaption verhältnismäßig harmlose Betriebsstörungen durch Fehlverhalten der Biologie als ernsthafte Prozeßeinbrüche fehlinterpretiert werden könnten. Solche Fehler können durch entsprechende vorträgliche Adaption der Mikroorganismen an dem jeweiligen Abwassertyp ausgeschaltet werden.

Um störende Einflüsse aufgrund von pH-Wertschwankungen im zulaufenden Wasser zu vermeiden, wird der By-pass-Strom zum Biosensor auf einen konstanten Wert geregelt. Im Normalfall kann man davon ausgehen, daß im Abwasser genügend Ammonium-Stickstoff vorkommt - den es anschließend zu nitrifizieren gilt - so daß die detektierte mikrobielle Aktivität hinreichend hoch ist. Es ist jedoch empfehlenswert, insb. dann, wenn die Konzentrationsverhältnisse im Zulauf z.T. starken Schwankungen unterworfen sind, den By-pass-Strom in definierter Weise mit Ammonium und essentiellen Nährstoffen zu versehen, um der Gefahr vorzubeugen, daß im Falle einer Stickstofflimitierung (z.B. aufgrund eines Produktionsausfalls des industriellen Abwasserlieferanten) die dadurch gegebene Meßwertminderung als Inhibierung interpretiert wird.

In Falle einer identifizierten toxischen Störung wird (wie bereits oben anhand von Figur 7 skizziert) über den Datenausgang des Rechnerinterface auf den Prozeß eingegriffen und die Zufuhr von Abwasser aus dem bis dahin auf einem Niveau-Minimum betriebenen Eingangsbehälter in den Belebungsreaktor gestoppt, das ankommende Abwasser im Eingangsbehälter oder in einem gesonderten Puffertank gesammelt. Bei einer volumenmäßigen Auslegung des Eingangsbehälters entsprechend der Hälfte der Verweilzeit im Bioreaktor (bei τ = 24 h in Belebungsbecken würde die Verweilzeit im Eingangsbehälter oder in einen gesonderten Puffertank 12 Stunden betragen) stünde dann genügend Zeit zur Verfügung, die Störung zu identifizieren und zu beseitigen, so daß das Abwasser erneut kontinuierlich durch die biologische Reinigungsanlage eingeleitet werden kann. Der intoxifizierte Biosensor würde zweckmäßigerweise innerhalb kürzester Zeit durch eine intakte (konservierte) Biologie erneut bestückt und für die weitere Prozeßüberwachung nach kurzer Adaptation eingesetzt werden.

Die Ausführung der vorgeschalteten Druchflußzelle (Vorprüfer) als Wirbelschichtreaktor bietet gegenüber den Festbetteinsatz dem Vorteil, daß auch partikuläre Bestandteile, welche bei einer physikalischen Vorbehandlung (z.B. Sedimentation) nicht erfaßt worden sind, problemlos durch dem Biosensor geschleust werden könnten. Alternativ zur Begasung mit Luft kommt die Verwendung von reinen Sauerstoff in Frage, wie in 15 angedeutet, um die aufgrund der geringen Sauerstofflöslichkeit in Wasser bestehende Stofftransportlimitierung zu überwinden und die immobilisierte mikrobielle Population bei maximaler Aktivität zu erhalten.

Bei einem anaeroben Prozeß resultiert durch den Abbau der Essigsäure ein pH-Anstieg, welcher entsprechend mittels einer geeigneten Säure kompensiert wird. Da gleichzeitig Biogas entsteht, kann dieses ebenfalls in einem auf der Basis der Wirbelschichttechnik realisierten Biosensor am einfachsten abgetrennt werden. Durch die signifikante Oberflächenvergrößerung gegenüber einem grobpartikulären Träger bei der Festbetttechnik wird die höchstmögliche Umsatzaktivität erreicht. Weil darüber hinaus Biokatalysatoren gegenüber chemischen Katalysatoren durch die Vermehrung gekennzeichnet sind, kann Überschußbiomasse im der Wirbelschichtvariante simultan mit dem Ablauf abgetrennt werden, wohingegen bei einem Festbettsystem die Biomasse größtenteils mechanisch zurückgehalten wird und die Verstopfungsanfälligkeit wächst.

## Patentansprüche

1. Verfahren zur kontinuierlichen Überwachung der volumenbezogenen Nitrifikationsaktivität in einem Abwasserstrom oder -becken,
**dadurch gekennzeichnet,**
daß man kontinuierlich einen Abwasserteilstrom von definierten pH-Wert mit bestimmter Verweilzeit durch eine als Bioreaktor ausgebildete, durch Zufuhr von pH-Korrekturmittellösung pH-kontrollierte Durchflußzelle schickt, die ggfs. mit angepaßter NH₄⁺-Standardlösung simultan oder alternativ gespeist wird und eine Nitrifikantenpopulation aufweist oder zusammen mit dem Abwasser erhält und daß man die zugesetzten pH-Korrekturmittelmengen fortlaufend mißt und im Signale der Nitrifikationsleistung unsetzt.

2. Verfahren nach Anspruch 1,
**dadurch gekennzeichnet,**
daß der Abwasserteilstrom von schlammführenden Reaktorablauf eines Abwasserreinigungsbeckens stammt, der eine Nitrifikantenpopulation enthält und mit gleicher Menge NH₄⁺-Standardlösung gemischt durch die Durchflußzelle geschickt wird.

3. Verfahren nach Anspruch 1,
**dadurch gekennzeichnet,**
daß der Abwasserteilstrom vom Abwasserzustrom zu einem Abwasserreinigungsbecken stammt und mit adäquater, insb. gleicher Menge Nitrifikanten enthaltenden Ablauf des Beckens gemischt durch die Durchflußzelle geschickt wird.

4. Verfahren nach Anspruch 1,
**dadurch gekennzeichnet,**
daß durch die Zelle alternierend eine Mischung von Abwasserzu- und -ablauf des Beckens bzw. eine Mischung von NH₄⁺-Standardlösung und Abwasserablauf des Beckens geschickt werden.

5. Verfahren nach einem der vorangehenden Ansprüche,
**dadurch gekennzeichnet,**
daß jeweils mit einer Verweilzeit von 0,5 bis 2 Stunden gearbeitet wird.

6. Verfahren nach einen der vorangehenden Ansprüche,
**dadurch gekennzeichnet,**
daß man als NH₄⁺-Standardlösung eine 80 mmol NH₄⁺/l - Lösung mit einem zum Abwasserteilstrom identischen pH-Wert zusetzt.

7. Verfahren nach einen der vorangehenden Ansprüche,
**dadurch gekennzeichnet,**
daß man als Korrekturmittellösung Na₂CO₃-Lösung verwendet.

8. Verfahren nach einen der vorangehenden Ansprüche,
**dadurch gekennzeichnet,**
daß in der Durchflußzelle zusätzlich die Trübung gemessen wird.

9. Anwendung des Verfahrens nach Anspruch 1 zur Steuerung einer nitrifizierenden Abwasserreinigungsstufe durch fortlaufende Überwachung der Nitrifikationsaktivität im Abwasserzulauf mit vorgegebenen, der Reinigungsstufe entsprechenden Mikroorganismen und Aufstau des Zulaufs in einem Misch- bzw. Speicherbehälter mit entsprechender zeitlicher Zulaufsperre zur Nitrifikationsstufe, wenn ein signifikanter Nitrifikationsaktivitätsabfall um ≧ 50 % innerhalb von 30 min signalisiert wird.

10. Als Bioreaktor ausgebildete Durchflußzelle für Bioaktivitätsbestimmungen mit geträgerter Biomasse, Meßsonden, insb. pH-Meßsonde und Dosierzulauf für Prüflösung und von Signal der pH-Meßsonde abhängigen Dosierzulauf für pH-Korrekturmittellösung, dessen ggfs. aufsummiertes Zugabemengen-Signal einer elektronischen Auswert-Einheit zugeleitet wird.

11. Durchflußzelle nach Anspruch 9 in Form eines Festbett- oder insb. Wirbelschichtreaktors mit biomassehaltigem porösen Granulat.

12. Durchflußzelle nach Anspruch 11,
**gekennzeichnet durch**
poröses Sinterglas als poröses Granulat.

13. Nitrifikationsstufe einer Abwasserreinigungsanlage mit einer gesondert Mikroorganismen aufweisenden oder erhaltenden Durchflußzelle mach einem der Ansprüche 10 bis 12 in der Zulaufstrecke zum Nitrifikationsreaktor zur Bewertung von Ammonium- und ggfs. Hemmstoffgehalt des zugeführten Rohwassers und einer Durchflußzelle nach Anspruch 10 in der Ablaufstrecke des Nitrifikationsreaktors zur Bewertung der Nitrifikationsaktivität der Reaktorbiomasse.
